(19) **Europäisches Patentamt**
**European Patent Office**
**Office européen des brevets**

(11) **EP 1 059 528 B1**

(12) **EUROPÄISCHE PATENTSCHRIFT**

(45) Veröffentlichungstag und Bekanntmachung des Hinweises auf die Patenterteilung:
**10.11.2010 Patentblatt 2010/45**

(51) Int Cl.:
***G01N 27/414*** *(2006.01)*

(21) Anmeldenummer: **00111575.7**

(22) Anmeldetag: **30.05.2000**

(54) **Gassensor nach dem Prinzip der Austrittsarbeitsmessung**

Gassensor using the principle of workfunction

Capteur de gaz sur le principe du potentiel d'extraction

(84) Benannte Vertragsstaaten:
**DE ES FR GB IT**

(30) Priorität: **11.06.1999 DE 19926747**

(43) Veröffentlichungstag der Anmeldung:
**13.12.2000 Patentblatt 2000/50**

(73) Patentinhaber: **Micronas GmbH**
**79108 Freiburg i. Br. (DE)**

(72) Erfinder:
 • **Ostrick, Bernhard**
  **81541 München (DE)**
 • **Fleischer, Maximilian, Dr.**
  **85635 Höhenkirchen (DE)**
 • **Pohle, Roland**
  **85570 Herdweg (DE)**
 • **Meixner, Hans, Prof.-Dr.**
  **85540 Haar (DE)**

(74) Vertreter: **Koch Müller**
**Patentanwaltsgesellschaft mbH**
**Maaßstraße 32/1**
**69123 Heidelberg (DE)**

(56) Entgegenhaltungen:
DE-A- 4 239 319   DE-A- 4 333 875
GB-A- 2 202 948   US-A- 4 411 741

• FUCHS A ET AL: "Room temperature ozone sensing with KI layers integrated in HSGFET gas sensors" SENSORS AND ACTUATORS B, ELSEVIER SEQUOIA S.A., LAUSANNE, CH, Bd. 48, Nr. 1-3, 30. Mai 1998 (1998-05-30), Seiten 296-299, XP004147354 ISSN: 0925-4005
• TIAN-HONG ZHANG ET AL: "TEMPERATURE-CONTROLLED KELVIN MICROPROBE" SENSORS AND ACTUATORS B, ELSEVIER SEQUOIA S.A., LAUSANNE, CH, Bd. B12, Nr. 3, 15. April 1993 (1993-04-15), Seiten 175-180, XP000397508 ISSN: 0925-4005
• DATABASE WPI Section Ch, Week 198243 Derwent Publications Ltd., London, GB; Class E35, AN 1982-92383E XP002219070 -& SU 892 655 A (LENGD AVIAT EQUIP), 23. Dezember 1981 (1981-12-23)
• SCHIERBAUM K D ET AL: "Defect structure and sensing mechanism of SnO2 gas sensors: Comparative electrical and spectroscopic studies" SOLID STATE IONICS, NORTH HOLLAND PUB. COMPANY. AMSTERDAM; NL, NL LNKD- DOI:10.1016/0167-2738(88)90432-8, vol. 28-30, 1 September 1988 (1988-09-01), pages 1631-1636, XP024682750 ISSN: 0167-2738 [retrieved on 1988-09-01]
• DOLL T ET AL: "Room temperature ozone sensing with conductivity and work function sensors based on indium oxide" 1997 INTERNATIONAL CONFERENCE ON SOLID-STATE SENSORS AND ACTUATORS. DIGEST OF TECHNICAL PAPERS. TRANSDUCERS 97. CHICAGO, IL, JUNE 16 - 19, 1997. SESSIONS 3A1 - 4D3. PAPERS NO. 3A1.01 - 4D3.14P; [INTERNATIONAL CONFERENCE ON SOLID-STATE SENSORS AND ACT, vol. 1, 16 June 1997 (1997-06-16), pages 577-580, XP010240542 ISBN: 978-0-7803-3829-6

EP 1 059 528 B1

**Beschreibung**

Die Erfindung betrifft einen Gassensor und ein Verfahren zur Bestimmung von Ammoniak und/oder Ammoniak-Derivaten nach dem Prinzip der Austrittsarbeitsmessung.

Ammoniak ($NH_3$) ist ein wasserlösliches, farbloses Gas, welches sich bei einem Anteil von 5 ppm (ppm = "parts per million" = Teile pro einer Million) durch einen stechenden Geruch bemerkbar macht und zwischen 20 ppm und 50 ppm die Atemwege und die Augen reizt. Die kurzzeitige Belastungsgrenze liegt bei 35 ppm.

Bei den Ammoniak-Derivaten sind ein oder mehrere Wasserstoff-Moleküle des Ammoniak durch ein oder mehrere CH-Gruppen ausgetauscht. Zu den Ammoniak-Derivaten gehören Amine wie beispielsweise Methylamin.

Ein Feldeffekt-Transistor (FET) als Gassensor (GasFET) ist in verschiedenen Ausführungsformen bekannt. Die Gassensitivität der GasFETs beruht darauf, daß der am GasFET meßbare Strom von einer Austrittsarbeit einer auf einer Oberfläche angebrachten gassensitiven Schicht abhängt. Ändert sich die Austrittsarbeit aufgrund einer Anwesenheit eines zu detektierenden Gases, so ändert sich auch der gemessene Strom.

Aus T. Doll et al.: "Ein Baukastensystem aus hybriden GasFET-Modulen", ITG-Fachbericht 126: Sensoren - Technologie und Anwendung, Seiten 465 bis 470, ist es bekannt, einen Feldeffekt-Transistor mit Luftspalt (Suspended Gate FET = SG-FET) einzusetzen, beispielsweise zur Wasserstoff-Detektion.

Ein weiterer SG-FET zur Gasdetektion ist in Matthias Peschke: "Wirkungsweise und Technologie von gassensitiven Suspended Gate Feldeffekt-Transistoren mit chemisch aktiven Zinnoxidschichten", Dissertation vom 4.7.90, Universität der Bundeswehr München, offenbart.

In DE 43 33 875 C2 wird ein kapazitiv gesteuerter Feldeffekt-Transistor beschrieben (Capacitive Controlled Field Effect Transistor = CC-FET). Eine Zusammensetzung einer gasempfindlichen Schicht ist nicht weiter angegeben.

Ein weiterer CC-FET ist beschrieben in Z. Gergintschew et al.: "The capacitively controlled field effect transistor (CC-FET) as a new low power gas sensor", Sensors and Actuators B 35-36 (1996) 285 - 289. Dieser CC-FET ist mit einer gassensitiven Schicht aus Pt, Pd oder Metalloxiden wie $SnO_2$ oder $Ga_2O_3$ ausgestattet. Dieser Sensor kann zur Detektion von $H_2$ und $NH_3$ verwendet werden.

Die Änderung der Austrittsarbeit kann mittels der KelvinMethode ("vibrating capacitor method") gemessen werden.

In K. Besocke und S. Berger, "Piezoelectric driven Kelvin probe for contact potential difference studies", Rev. Sci. Instrum., vol. 47, no. 7, July 1976, Seiten 840 bis 842, wird dabei ein Schwinger (z. B. ein Metallplättchen) mittels eines Piezoelementes zu Schwingungen angeregt. Am Ende des Schwingers ist eine Kondensatorplatte angebracht, welche einer zweiten Kondensatorplatte gegenüberliegt. Beide Kondensatorplatten weisen unterschiedliche Materialien auf, so daß am Kondensator eine Austrittsarbeits-Differenz (Kontaktpotential-Differenz) anliegt.

Wegen der Vibration des Schwingers, und damit einer Kondensatorplatte, wird ein Wechselstrom erzeugt, welcher gemessen werden kann und der ein Maß für die Austrittsarbeits-Differenz darstellt.

Eine weitere Ausführung der Kelvinmethode zur Messung der Austrittsarbeit ist in P.L. Bergstrom et al., TRANSDUCERS '95 • EUROSENSORS IX, The 8th International Conference on Solid-State Sensors and Actuators, and Eurosensors IX, Stockholm, Sweden, June 25 - 29, 1995, Seiten 993 bis 996, beschrieben. Hier ist die schwingende Seite des Kondensators als Membran ausgeführt.

Zur Ammoniak-Bestimmung sind auch Sensorsysteme auf der Basis von optischen Systemen bekannt, bei denen eine ammoniaksensitive Substanz durch ihre Farbänderung ausgelesen wird, und von elektrochemischen Zellen. • Elektrochemische Zellen und optische Systeme sind vergleichsweise teuer, und eine elektrochemischen Zelle besitzt nur eine begrenzte Lebensdauer von typischerweise einem Jahr.

Auch sind Gassensoren mit einer gassensitiven Schicht aus halbleitendem Metalloxid, z. B. GaAs, bekannt. Ein solcher Gassensor besitzt meist aufgrund seiner hohen Arbeitstemperatur von über 200°C eine erhöhte Heizleistung und ist daher beispielsweise nicht für Handmeßgeräte geeignet.

Auch sind Gassensoren auf der Basis einer elektrochemischen Zelle oder eines halbleitenden Metalloxids querempfindlich gegen eine Vielzahl anderer Gase. Der von diesen Zellen abdeckbare Meßbereich ist für viele Anwendungen zu ungenau (30 ppm bis 300 ppm).

Zur Ammoniak-Detektion sind auch Systeme auf der Basis von massensensitiven Quarzschwingern (Quarz Microbalance = QMB) bekannt. Dabei handelt es sich um billige Sensorelemente, für die jedoch eine aufwendige Signalverarbeitung (Frequenzanalyse) nötig ist. Zudem sind bei dieser Methode der Ammoniak-Detektion Sensorarrays von bis zu 5 Sensoren nötig, damit in erster Linie die Querempfindlichkeit gegenüber Wasser ausgeschaltet werden kann: Bei typischen Detektionsbedingungen von 3 ppm bis 30 ppm Ammoniak und 25 % bis 30 % Feuchte zeigt die Feuchtevariation (25 % bis 30% Feuchte) einen ähnlich hohen Meßeffekt wie die Variation der Ammoniakkonzentration (3 ppm bis 30 ppm Ammoniak).

Als Stand der Technik betreffend Gassensoren, insbesondere zur Ammoniak-Detektion, sind die GB 2 202 948, die US 4,411,741, die DE 42 39 319, die WPI Database, Section CH, Week 198243 der Derwent Publications Ltd., Class E35, AN sowie die Artikel "Temperature-controlled Kelvin microprobe" by Tian-Hong Zhang et al. (Sensors and Actuators, B Chemical, Bd. B12 No. 3, Seiten 175-180; "Room temperature ozone sensing with KI layers integrated in HSGFET gas sensors" by A. Fuchs et al. (Sensors and Actuators, B, Bad. 48, Nr. 1-3, Seiten 296-299); "Defect structure and sensing mechanism of SnO2 gas sensors: Comparative electrical and spectroscopic studies" by Schierbaum et al., Solid State Ionics 28-30, Seiten 1631-1636 and "Room temperature ozone sensing with conductivity and work function sensors based on Indium oxide" by Doll et al., Transducers '97, International Conference on Solid-State Sensors and Actuators," bekannt.

Es ist eine Aufgabe der vorliegenden Erfindung, eine Meßmethode zur Bestimmung von Ammoniak und/oder Ammoniak-Derivaten mit hoher Empfindlichkeit bereitzustellen.

Es ist eine weitere Aufgabe der vorliegenden Erfindung, eine Methode zur Bestimmung von Ammoniak und/ oder Ammoniak-Derivaten mit geringer Querempfindlichkeit bereitzustellen.

Eine weitere Aufgabe der vorliegenden Erfindung ist es, ein preiswertes und langlebiges System zur Bestimmung von Ammoniak und/oder Ammoniak-Derivaten bereitzustellen.

Es ist eine weitere Aufgabe der vorliegenden Erfindung, eine Methode zur Bestimmung von Ammoniak und/ oder Ammoniak-Derivaten mit geringer Leistungsaufnahme bereitzustellen.

Diese Aufgaben werden durch einen Gassensor gemäß des Patentanspruchs 1 gelöst. Vorteilhafte Ausgestaltungen sind den Unteransprüchen entnehmbar.

Dazu wird ein Gassensor verwendet, welcher eine gassensitive Schicht aufweist, die Titannitrid, Titanoxinitrid oder eine Mischung dieser Bestandteile enthält. Bei einer Beaufschlagung des Gassensors mit Ammoniak und/oder einem oder mehreren Ammoniak-Derivaten wird an der gassensitiven Schicht eine meßbare Änderung der Austrittsarbeit hervorgerufen. Die Änderung der Austrittsarbeit stellt ein Maß für eine Konzentrationsänderung von Ammoniak und/ oder Ammoniak-Derivat dar, sie ist mit bekannten Mitteln meßbar.

Der Begriff " Titannitrid, Titanoxinitrid oder eine Mischung dieser Bestandteile enthaltend" wird so verwendet, daß das Titannitrid, Titanoxinitrid oder die Mischung dieser Bestandteile entweder als Verbindung, z. B. in Form von Titannitrid (TiN) oder Titanoxinitrid ($TiO_xN_x$) oder als Komponente einer Mischverbindung vorliegen kann.

Titanoxid ist chemisch vergleichsweise stabil und mit einem Siebdruckverfahren aufbringbar, Titannitrid ist gut leitend, und die Eigenschaften von Titanoxinitrid sind gut durch die Menge von Sauerstoff und Stickstoff einstellbar.

Die Titanverbindungen können stöchiometrisch oder unstöchiometrisch vorliegen. Eingeschlossen ist ebenfalls eine Mischung verschiedenen Titanzustände, z. B. eine Schicht aus TiN mit einer oxidierten Oberfläche.

Eine Verwendung von Titannitrid, Titanoxinitrid oder einer Mischung dieser Bestandteile in der gassensitiven Schicht ergibt den Vorteil einer gesteigerten Sensivität des Gassensors in Bezug auf Ammoniak und Ammoniak-Derivate.

Weiterhin zeigt die gassensitive Schicht bei einer Verwendung von Titannitrid, Titanoxinitrid oder einer Mischung dieser Bestandteile kaum eine Querempfindlichkeit mit anderen Gaskomponenten.

Zudem sind Titannitrid, Titanoxinitrid oder eine Mischung dieser Bestandteile enthaltende gassensitive Schichten vergleichsweise leicht aufbringbar, so daß eine kostengünstige Herstellung des Gassensors erreichbar ist.

Die gassensitive Schicht kann als Dünnschicht oder Dickschicht vorliegen. Ein Aufbringen der Schicht ist nicht an eine bestimmte Methode gebunden und kann beispielsweise mechanisch (z.B. Siebdruckfilm), chemisch (z.B. CVD) oder physikalisch (z.B. Sputtern) geschehen.

Es ist vorteilhaft, wenn der Gassensor ein gassensitiver Feldeffekttransistor (GasFET) ist, weil dieser eine gut verstandene Möglichkeit zur Auslesung der Austrittsarbeit darstellt. Mögliche Bauformen des Gas-FETs sind beispielsweise HSG-FETs (= Hybrid Suspended Gate FET), HFC-FETs oder CC-FETs. Dabei liefert die Änderung der Austrittsarbeit einen zusätzlichen Beitrag zur Gatespannung, auch Einsatzspannung genannt, durch den sich ein Source-Drain-Strom ISD ändert. Insbesondere die Verwendung eines HSG-FETs wird als günstig angesehen, weil sich dabei eine einfache Abscheidung der gassensitiven Schicht in Form einer Titanverbindung im Bereich des Gates erlaubt. Dieser Sensoraufbau ist vorteilig klein, zeigt keine Leistungsaufwärme, ist leicht auswertbar und billig herstellbar.

Es wird eine gassensitive Schicht, insbesondere in Form einer TiN-Schicht, bevorzugt, die auf einem Gate des HSG-FETs aufgebracht wird.

Die gassensitive Schicht muß nicht dem zu detektierenden Gas direkt ausgesetzt sein. Vielmehr kann sie auch vom Gas durch eine Filter- oder Schutzschicht getrennt sein, welche schädliche (z. B. korrosive oder eine Querempfindlichkeit hervorrufende) Gaskomponenten abhält.

Der Gassensor zur Bestimmung von Ammoniak und Ammoniak-Derivaten, insbesondere Aminen, kann vorteilhaft angewendet werden bei extensiver Tierhaltung, beispielsweise für eine Belüftungssteuerung in Ställen, z.B. in der Schweinemast, Hühnermast oder in Großraumbüros. Bei der Tierhaltung sollen Änderungen des Ammoniak- oder Ammoniak-Derivat-Gehaltes typischerweise zwischen 10 ppm und 50 ppm detektiert werden. Dabei tritt die Luftfeuchte als hauptsächliches Störgas mit typischen Variationen zwischen 7000 ppm und 9000 ppm, entsprechend 25% bis 32% Luftfeuchte bei 23°C, auf. In diesem Bereich wird besonders eine Verwendung preiswerter Sensoren mit einer geringen Leistungsaufnahme angestrebt.

Ein weiteres bevorzugtes Anwendungsfeld ist die Überwachung einer Kühlanlage bzw. eines Kühlhauses mit einem auf Ammoniak basierenden Kühlkreislauf. Hierbei steht eine Detektion einer Leckage und eine Überwachung einer Geruchsbelastung im Vordergrund. Dies entspricht in der Praxis einer Bestimmung einer Ammoniak-Konzentration zwischen ca. 1 ppm und 5 ppm.

Zur Lecksuche, die nicht auf eine bestimmte Anwendung, wie in Kühlanlagen, beschränkt ist, ist ein tragbares Handmeßgerät, günstigerweise unter Verwendung von Sensoren ohne Leistungsaufnahme, vorteilhaft.

Auch ist, insbesondere bei einer Verwendung preiswerter Sensoren, bevorzugt ohne Leistungsaufnahme, eine Bestückung von Räumlichkeiten mit mehreren Sensoren vorteilhaft, weil so mittels einer ortsaufgelösten Messung ein Leck lokalisierbar ist.

In den folgenden Ausführungsbeispielen wird der Gassensor schematisch näher dargestellt.

Figur 1    zeigt einen Gassensor in Form eines Feldeffekt- Transistors,

Figur 2    zeigt eine Messung einer Änderung einer Austrittsar- beit $\Delta\phi$ gegen die Zeit t in Abhängigkeit vom anliegenden Ammoniak-Partialdrucks p,

Figur 3    zeigt eine Auftragung einer Änderung einer Aus- trittsarbeit $\Delta\phi$ gegen den anliegenden Ammoniak- Partialdruck p

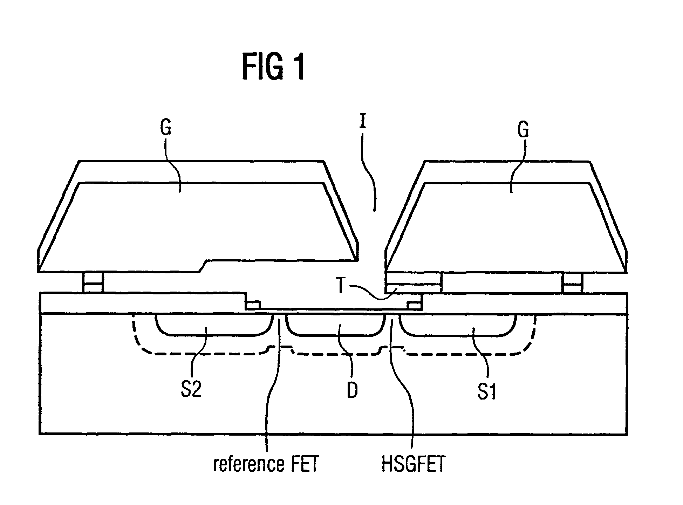

Figur 1 zeigt als Schnittdarstellung in Seitenansicht einen gassensitiven Feldeffekttransistor (FET) mit teilweise hybridem Aufbau (HSG-FET) mit Silizium-Micromachining-Gate.

In einem Silizium-Substrat sind zwei Quellen ("Sources") S1,S2 eingebracht sowie eine Senke ("Drain") D. Die Bereiche S1, S2 und D sowie die Kanäle dazwischen sind mittels $Si_3N_4$ passiviert.

Beide Kanäle zwischen Source S1,S2 und Drain D sind durch ein Silizium-Gate G überdacht, das durch einen Luftspalt abgehoben ist. Dadurch ergibt sich eine zweifache FET-Konfiguration, wobei die erste Konfiguration, das eigentliche GasFET, ein Gate G, das im Bereich zwischen dem Source S1 und dem Drain D mit einer gassensitiven Schicht T, hier einer TiN-Dünnschicht, beschichtet ist, umfaßt (rechtes FET). Das zweite, unbeschichtete, System Source S2 - Gate G - Drain D stellt einen Referenz-FET zur Kompensation des Temperaturgangs dar (linkes FET).

Zur Gasdetektion, d. h. zur Bestimmung der Anwesenheit oder zur Bestimmung einer Konzentration bzw. Konzentrationsänderung, von Ammoniak und/oder Ammoniak-Derivat in einem Gas, gelangt Gas durch einen Gaseinlaß I an die FETs. Dadurch wird die Potentialdifferenz zwischen der gassensitiven Schicht T und der Passivierungsschicht aus $Si_3N_4$, und damit einhergehend auch die Einsatzspannung des Transistors geändert. Über ein Messung der Änderung des Stroms zwischen Source S1 und Drain D ist ein Vorhandensein oder eine Konzentration von Ammoniak und/oder Ammoniak-Derivat meßbar.

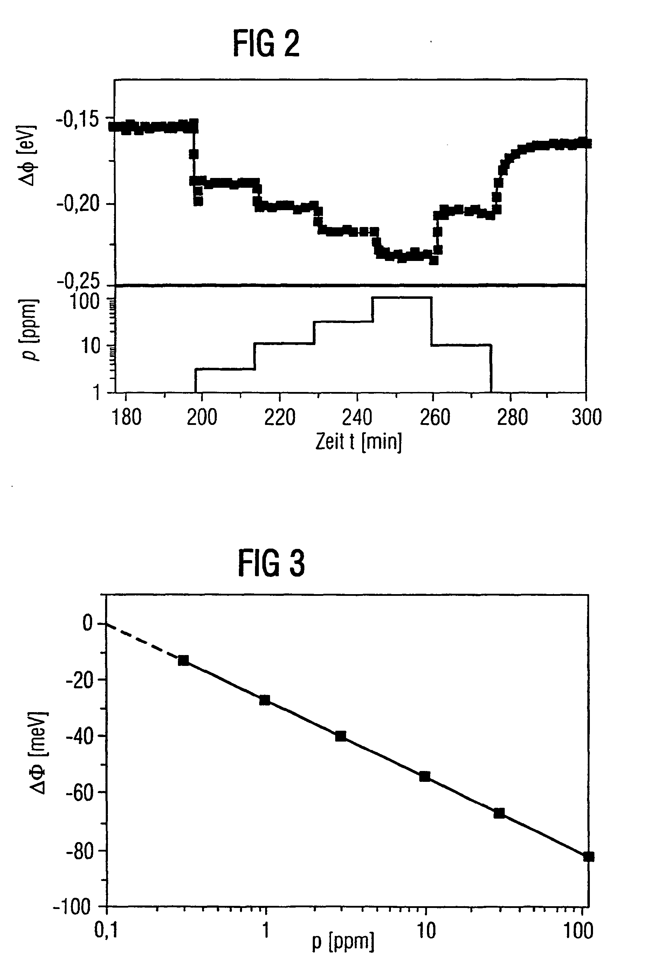

Figur 2 zeigt eine Auftragung einer Änderung der Austrittsarbeit $\Delta\phi$ in eV gegen eine Meßzeit t in min (oberes Diagramm), gemessen mit einer Kelvinsonde. Über die gleiche Zeitachse ist ein Partialdruck p von Ammoniak in ppm, der an der Kelvinsonde anliegt, logarithmisch aufgetragen (unteres Diagramm).

Die verwendete Kelvinsonde entspricht im wesentlichen dem Aufbau aus K. Besocke and S. Berger (siehe oben). Die am piezoelektrisch angetriebenen Schwinger angebrachte Kondensatorplatte besteht aus einem feinen Goldnetz. Die stationäre Kondensatorplatte besteht aus TiN. Der Partialdruck p des Ammoniak wird zwischen 0 ppm und 100 ppm bei 23°C und ca. 25 % relativer Feuchte variiert.

Diese Meßkurve zeigt deutlich, daß schon eine geringe Änderung des Partialdrucks p des Ammoniak am TiN eine signifikante Änderung der Austrittsarbeit $\Delta\phi$ bewirkt. Zudem ergibt sich eine sehr schnelle Ansprechzeit im Bereich < 1 min und eine Abklingzeit im Bereich von 2 min bis 3 min.

Figur 3 zeigt eine Auftragung der Änderung der Austrittsarbeit $\Delta\phi$ in meV gegen den Partialdruck p des Ammoniak in ppm bei logarithmischer Skala der Abszisse für einen Meßaufbau wie er in Figur 2 beschrieben ist.

Das Diagramm zeigt, daß eine Änderung der Austrittsarbeit $\Delta\Phi$ von ca. 30 mV pro Dekade Ammoniak-Partialdruck p auftritt. Das Detektionslimit liegt bei ca. 100 ppb (ppb = part per billion = 1 Teil auf eine Milliarde Teile), entsprechend

$$\Delta\Phi(p(t1)) - \Delta\Phi(p(t2)) = 30 \text{ meV} \cdot \log(p(t2)/p(t1)),$$

für p > 100 ppb, wobei p(t1) die Konzentration von Ammoniak zu einem Zeitpunkt t1 und p(t2) die Konzentration von Ammoniak zu einem Zeitpunkt t2 bezeichnet. Selbstverständlich ist die Methode nicht auf diese Meßbereiche beschränkt.

Dieses Diagramm zeigt nochmals deutlich die außerordentliche Sensitivität des TiN in Bezug auf eine Änderung der am TiN anliegenden Ammoniakkonzentration sowie die sehr gute Möglichkeit einer quantitativen Auswertung einer Konzentrationsänderung von Ammoniak. Eine Verwendung von TiN ist daher z. B. für die oben geschriebenen Anwendungen im Meßbereich von 100 ppb bis 3 ppb und von 1 ppm bis 10 ppm geeignet. Dies gilt sowohl für ein Vorhandensein einer TiN-Oberfläche in einer Kelvinsonde als auch für andere Sensorarten, z. B. einen GasFET.

Darüber hinaus zeigt ein Gassensor mit einer TiN-Oberfläche kaum Querempfindlichkeiten, unter anderem auf eine zusätzliche Beigabe von 100 ppb $NO_2$, 3000 ppm $CO_2$, 30 ppm CO oder 10 ppm Aceton, Methanol, Toluol, Wasserstoff oder Ethen. Auf diese Zusatzstoffe reagiert der Gassensor bei Raumtemperatur nicht.

Auch Feuchte zeigt einen nur geringen Einfluß von 3 meV bei einem aufgesputterten Film und von 10 meV bei einem mittels Siebdruck aufgebrachten Film bei einer Änderung der Luftfeuchte zwischen 25% und 35%. Es ist zudem möglich, daß ein Teil der Änderung der Austrittsarbeit bei dem Siebdruckfilm auf zunächst noch verbleibende Reste eines Bindemittels zurückzuführen ist.

## Patentansprüche

1. Gassensor nach dem Prinzip der Austrittsarbeitsmessung zur Bestimmung von Ammoniak und/oder Ammoniak-Derivaten, aufweisend
eine gassensitive Schicht (T),
**dadurch gekennzeichnet, daß** die gassensitive Schicht (T) Titannitrid, Titanoxinitrid oder eine Mischung dieser Bestandteile enthält.

2. Gassensor nach Anspruch 1, bei dem
das Titannitrid, Titanoxinitrid oder eine Mischung dieser Bestandteile als eine Komponente einer Mischverbindung vorliegt.

3. Gassensor nach einem der vorhergehenden Ansprüche, bei dem die gassensitive Schicht (T) als Dünnschicht oder Dickschicht vorliegt.

4. Gassensor nach einem der vorhergehenden Ansprüche, bei dem die gassensitive Schicht (T) ein Teil einer Kelvinsonde ist.

5. Gassensor nach einem der Ansprüche 1 bis 3, bei dem die gassensitive Schicht (T) ein Teil eines FeldeffektTransistors ist.

6. Gassensor nach Anspruch 5, bei dem
der Feldeffekt-Transistor ein HSG-FET ist.

7. Gassensor nach Ansprüche 6, bei dem
die gassensitive Schicht (T) auf einem Gate aufgebracht ist.

8. Gassensor nach Anspruch 5, bei dem
der Feldeffekt-Transistor ein CC-FET oder ein HFC-FET ist.

9. Gassensor nach einem der vorhergehenden Ansprüche, der in einem Sensorsystem zur Überwachung einer Konzentration von Ammoniak und/oder Ammoniak-Derivaten in der Tierhaltung oder an einer Kühlanlage einsetzbar ist.

## Claims

1. Gas sensor according to the principle of output work measurement for determination of ammonia and/or ammonia derivatives, comprising a gas-sensitive layer (T), **characterised in that** the gas-sensitive layer (T) contains titanium nitride, titanium oxynitride or a mixture of these components.

2. Gas sensor according to claim 1, in which the titanium nitride, titanium oxynitride or a mixture of these components is present as a component of a compound.

3. Gas sensor according to any one of the preceding claims, in which the gas-sensitive layer (T) is present as a thin layer or a thick layer.

4. Gas sensor according to any one of the preceding claims, in which the gas-sensitive layer (T) is part of a kelvin probe.

5. Gas sensor according to any one of claims 1 to 3, in which the gas-sensitive layer (T) is part of a field effect transistor.

**6.** Gas sensor according to claim 5, in which the field effect transistor is an HSG-FET.

**7.** Gas sensor according to claim 6, in which the gas-sensitive layer (T) is applied to a gate.

**8.** Gas sensor according to claim 5, in which the field effect transistor is a CC-FET or an HFT-FET.

**9.** Gas sensor according to any one of the preceding claims, which is usable in a sensor system for monitoring a concentration of ammonia and/or ammonia derivatives in the keeping of animals or in a cooling plant.

**Revendications**

**1.** Capteur de gaz selon le principe du potentiel d'extraction pour le dosage d'ammoniac ou de dérivés d'ammoniac comprenant une couche (T) sensible au gaz **caractérisé en ce que** la couche (T) sensible au gaz contient du nitrure de titane, de l'oxynitrure de titane ou un mélange de ces composants.

**2.** Capteur de gaz selon la revendication 1 dans lequel le nitrure de titane, l'oxyniture de titane ou un mélange de ces composants se présente comme un constituant d'un mélange de liaison.

**3.** Capteur de gaz selon l'une des revendications précédentes dans lequel la couche (T) sensible au gaz se présente comme une couche mince ou une couche épaisse.

**4.** Capteur de gaz selon l'une des revendications précédentes dans lequel la couche (T) sensible au gaz constitue une partie d'une sonde Kelvin.

**5.** Capteur de gaz selon l'une des revendications 1 à 3 dans lequel la couche (T) sensible au gaz constitue une partie d'un transistor à effet de champ.

**6.** Capteur de gaz selon la revendication 5 dans lequel le transistor à effet de champ est un transistor à effet de champ HSG.

**7.** Capteur de gaz selon la revendication 6 dans lequel la couche (T) sensible au gaz est appliquée sur une grille.

**8.** Capteur de gaz selon la revendication 5 dans lequel le transistor à effet de champ est un transistor à effet de champ CC ou HFC.

**9.** Capteur de gaz selon l'une des revendications précédentes **caractérisé en ce qu'**il est intégré dans un système de capteur pour la surveillance de la concentration en ammoniac et/ou en dérivés d'ammoniac dans le domaine de l'entretien des animaux ou dans une installation de réfrigération.

# FIG 1

# FIG 2

# FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4333875 C2
- GB 2202948 A
- US 4411741 A
- DE 4239319

**In der Beschreibung aufgeführte Nicht-Patentliteratur**

- **T. Doll et al.** Ein Baukastensystem aus hybriden GasFET-Modulen. *ITG-Fachbericht 126: Sensoren - Technologie und Anwendung,* 465-470
- **Z. Gergintschew et al.** The capacitively controlled field effect transistor (CC-FET) as a new low power gas sensor. *Sensors and Actuators B,* 1996, vol. 35-36, 285-289
- **K. Besocke ; S. Berger.** Piezoelectric driven Kelvin probe for contact potential difference studies. *Rev. Sci. Instrum.,* Juli 1976, vol. 47 (7), 840-842
- **P.L. Bergstrom et al.** *TRANSDUCERS '95 • EUROSENSORS IX, The 8th International Conference on Solid-State Sensors and Actuators, and Eurosensors IX,* 25. Juni 1995, 993-996
- **Tian-Hong Zhang et al.** Temperature-controlled Kelvin microprobe. *Sensors and Actuators, B Chemical,* vol. B12 (3), 175-180
- **A. Fuchs et al.** Room temperature ozone sensing with KI layers integrated in HSGFET gas sensors. *Sensors and Actuators, B,* vol. 48 (1-3), 296-299
- **Schierbaum et al.** Defect structure and sensing mechanism of SnO2 gas sensors: Comparative electrical and spectroscopic studies. *Solid State Ionics,* vol. 28-30, 1631-1636
- **Doll et al.** Room temperature ozone sensing with conductivity and work function sensors based on Indium oxide. *Transducers '97, International Conference on Solid-State Sensors and Actuators*